# EUROPEAN PATENT APPLICATION

(11) **EP 2 586 375 A1**
(43) Date of publication of application: **01.05.2013**
(21) Application number: 12184071.4
(22) Date of filing: 12.09.2012
(51) Int. Cl.: A61B 6/10, A61B 6/04, A61B 6/03, G01T 1/164

(54) **Radiographic apparatus for breast examination**

(30) Priority: 26.10.2011 JP 2011235026
(71) Applicant: Shimadzu Corporation, Kyoto-shi, Kyoto 604 (JP)
(72) Inventor: Tonami, Hiromichi, Kyoto-shi,, Kyoto 604-8511, (JP)
(74) Representative: Kilian Kilian & Partner

(57) **Abstract**

A radiographic apparatus for breast examination is provided, the apparatus including a shielding plate that reliably shields radiation derived from the trunk and is not obstructive upon inserting a breast into a detector ring. The shield plate has a thickness that varies depending on portions thereof. That is, the shield plate has a thick portion that is thick in a central axis direction of the detector ring, and a thin portion that is thin in the central axis direction. Such thick portion of the shield plate can absorb radiation reliably that is emitted a lot from a heart of a subject. Moreover, in the thin portion, a distance between the subject and the detector ring can be closer. Therefore, the apparatus can obtain a clear tomographic image containing an entire image of the breast.

## Description

### BACKGROUND OF THE INVENTION

### 1. Field of the Invention

This invention relates to a radiographic apparatus for breast examination that detects annihilation radiation pairs emitted from a subject and images radiopharmaceutical distribution in the subject. Particularly, this invention relates to a radiographic apparatus for breast examination that is used for cancer screening.

### 2. Description of the Related Art

Medical institutions are equipped with a radiographic apparatus that allows imaging of radiopharmaceutical distribution. Description will be given of a specific configuration of such radiographic apparatus. The conventional radiographic apparatus includes a radiation ring having radiation detectors arranged circularly for detecting radiation. The detector ring detects a pair of radiation (an annihilation radiation-pair), the pair of radiation with opposite directions to each other being emitted from inside of a subject. See, for example, Japanese Patent No. JP-3828195.

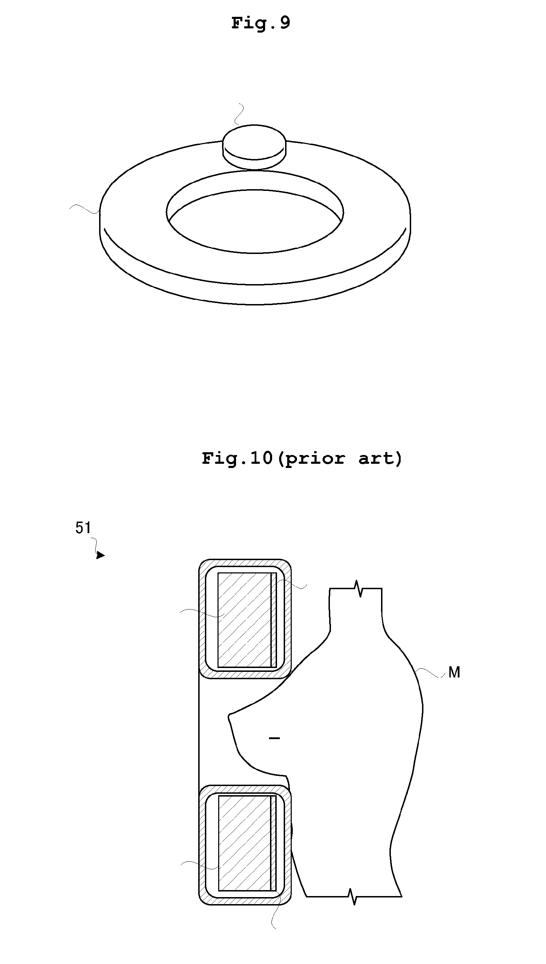

Examples of such radiographic apparatus include a radiographic apparatus for breast examination. Description will be given in detail of the radiographic apparatus for breast examination. Figure 10 illustrates a conventional radiographic apparatus for breast examination. In the conventional radiographic apparatus 51 for breast examination, one breast B of a subject M is inserted into a detector ring 62 upon screening. Under this state, the detector ring 62 detects annihilation radiation-pairs emitted from the subject M.

The detector ring 62 identifies an emission source of the annihilation radiation pairs emitted from the breast B. Then a distribution map of radiopharmaceutical is generated in accordance with positional information of the source. Since radiopharmaceutical tends to accumulate in cancer tissue rather than normal tissue, interpreting the distribution map of radiopharmaceutical can achieve screening of breast cancer.

The detector ring 62 includes a shield plate 63 on a side where the breast is inserted. When radiation from a trunk enters into the detector ring 62, the breast B cannot be imaged successfully. Thus the shield plate 63 is provided to avoid entering of radiation from the trunk into the detector ring 62.

The conventional construction, however, has the following drawbacks.

Specifically, the shield plate is obstructive upon introducing the breast into the detector ring. The conventional construction has such drawback. The shield plate needs to absorb radiation from the trunk of the subject with reliability. For this purpose, the shield plate needs to have a certain thickness.

On the other hand, it becomes more difficult to insert the breast into the detector ring 62 deeply as the shield plate 63 becomes thicker. When the breast cannot be inserted into the detector ring 62 satisfactorily, this increases a possibility that an image of the breast cancer cannot be contained in a tomographic image to be obtained. That is because a joint of the breast where a breast cancer tends to occur cannot be examined when the breast is not inserted into the detector ring 62 deeply.

When the shield plate 63 is made thinner so as to insert the breast into the detector ring 62 deeply, radiation emitted from the trunk of the subject towards the detector ring 62 cannot be shielded satisfactorily. Then, the detector ring 62 detects radiation in the breast that is not derived from the radiopharmaceutical, which causes difficulty in obtaining a clear tomographic image.

### SUMMARY

This invention has been made regarding the state of the art noted above, and its object is to provide a radiographic apparatus for breast examination. The apparatus includes a shield plate that reliably shields radiation derived from the trunk and is not obstructive upon inserting a breast into a detector ring.

Additional features of the invention will be set forth in the description which follows, and in part will be apparent from the description, or may be learned by practice of the invention.

This invention is constituted as stated below to achieve the above object.

One example of this invention discloses a radiographic apparatus for breast examination. The radiographic apparatus includes a detector ring having detectors for detecting radiation being arranged in an arc shape, and a shield plate for absorbing radiation that is provided as to cover a side face of the detector ring perpendicular to a central axis of the detector ring. The shield plate has a thick portion that is thick in a central axis direction, and a thin portion that is thin in the central axis direction.

The shield plate according to the example of this invention has a thickness that varies depending on portions thereof. That is, the shield plate has a thick portion that is thick in the central axis direction of the detector ring, and a thin portion that is thin in the central axis direction of the detector ring. In such thick portion of the shield plate, radiation emitted a lot from a heart of the subject, etc. can be absorbed reliably. Thereby radiation emitted from a portion, such as a heart, where radiopharmaceutical has been accumulated is absorbed into the thick portion of the shield plate and thus does not reach the detector ring. On the other hand, in the thin portion, a distance between the subject and the detector ring is closer. Thus, a breast can be contacted more tightly to the detector ring. Thereby, radiation emitted from the breast reaches the detector ring reliably. As mentioned above, the radiographic apparatus according to the example of this invention can prevent radiation emitted from the trunk of the subject from entering into the detector ring. Moreover, the apparatus according to the example of this invention can reliably insert the breast into the detector ring and conduct a reliable diagnosis of the breast. Therefore, the apparatus according to the example of this invention can achieve suppression of a false image, thereby obtaining a clear tomographic image containing an entire image of the breast.

Moreover, in the radiographic apparatus for breast examination according to the example of this invention, it is more desirable that one end face of the shield plate on a subject side is planar.

The foregoing constitution is a more specific construction of the radiographic apparatus for breast examination according to this invention. The shield plate has one end face on the subject side that is planar with no projection and depression. Thus the subject does not contact to projections and depressions, which can achieve a diagnosis without causing any pain to the subject.

Moreover, in the radiographic apparatus for breast examination according to the example of this invention, it is more desirable that the shield plate is arranged in a direction where the thick portion approaches the heart of the subject upon screening.

The foregoing constitution is a more specific construction of the radiographic apparatus for breast examination according to this invention. When the shield plate is arranged in the direction where the thick portion approaches the heart of the subject upon screening, a clear tomographic image can be obtained with higher reliability. Here, radiopharmaceutical tends to be accumulated in the heart of the subject. Consequently, strong radiation is emitted from the heart. With the construction, radiation from the heart can be absorbed in the thick portion of the shield plate reliably. Thereby radiography of the breast can be conducted with low noise.

Moreover, the radiographic apparatus for breast examination according to the example of this invention desirably includes a rotating device for rotating the shield plate about the central axis, and a rotation control device for controlling the rotating device.

The foregoing constitution is a more specific construction of the radiographic apparatus for breast examination according to this invention. The shield plate can rotate, and accordingly the thick portion can be moved to a portion in the trunk of the subject where much radiation is generated. Moreover, the thick portion of the shield plate can be shifted upon screening a right breast and a left breast of the subject. Such configuration can cause the shield plate to be shifted in accordance with a position of the trunk of the subject that varies for every screening.

The shield plate in this invention has a thickness that varies depending on portions thereof. That is, the shield plate in this invention has the thick portion that is thick in the central axis direction of the detector ring, and the thin portion that is thin in the central axis direction of the detector ring. In such thick portion of the shield plate, radiation emitted a lot from the heart of the subject, etc. can be absorbed reliably. In the thin portion, a distance between the subject and the detector ring can be closer. Therefore, the apparatus according to this invention can obtain a clear tomographic image containing an entire image of the breast.

It is to be understood that both the foregoing general description and the following detailed description are exemplary and explanatory and are intended to provide further explanation of the invention as claimed.

### BRIEF DESCRIPTION OF THE DRAWINGS

The accompanying drawings, which are included to provide a further understanding of the invention and are incorporated in and constitute a part of this specification, illustrate embodiments of the invention, and together with the description serve to explain the principles of the invention.
Figure 1 is a functional block diagram illustrating a radiation tomographic apparatus according to one example.
Figure 2 is a perspective view illustrating a shield plate according to the example.
Figure 3 is a schematic view illustrating a positional relationship between the shield plate and a detector ring according to the example.
Figures 4 and 5 are schematic views each illustrating the shield plate according to the example.
Figure 6 is a plan view illustrating the detector ring according to the example.
Figure 7 is a perspective view illustrating a radiation detector according to the example.
Figure 8 is a functional block diagram illustrating one modification according to this invention.
Figure 9 is a perspective view illustrating the modification according to this invention.
Figure 10 is a sectional view illustrating a conventional radiographic apparatus.

### DESCRIPTION OF THE PREFERRED EMBODIMENTS

The invention is described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure is thorough, and will fully convey the scope of the invention to those skilled in the art. In the drawings, the size and relative sizes of layers and regions may be exaggerated for clarity. Like reference numerals in the drawings denote like elements.

Examples of a radiation tomographic apparatus according to this invention will be described hereinafter with reference to the drawings. A gamma-ray in Example 1 is one example of radiation in this invention. Here, Example 1 has a construction of a mammography apparatus for breast examination. That is, the radiographic apparatus in Example 1 images radiopharmaceutical distributed in a breast B, and generates a tomographic image.

### <Whole Construction of Radiographic Apparatus>

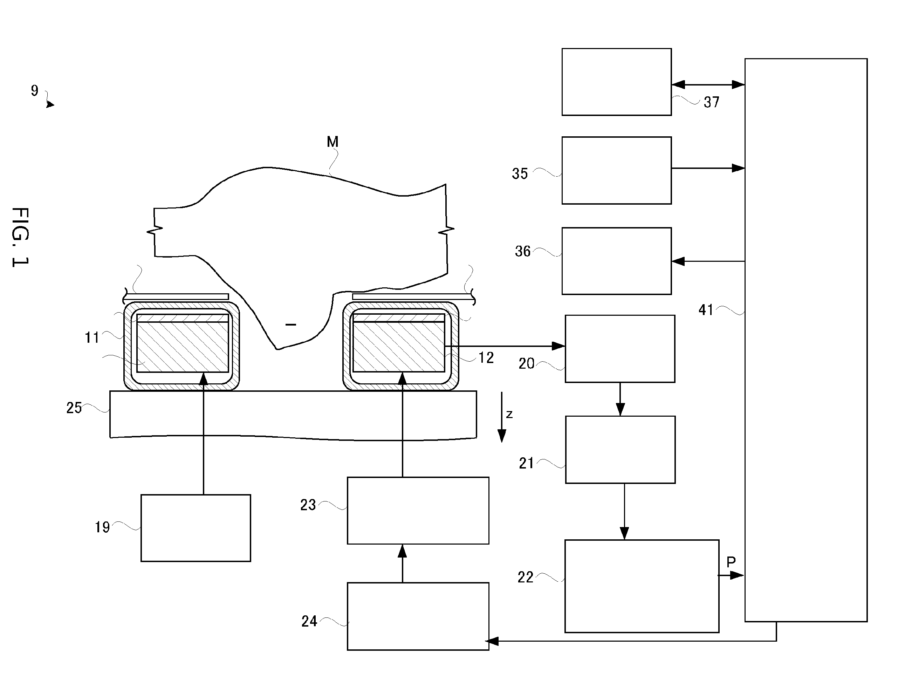

Figure 1 is a functional block diagram showing a concrete construction of a radiographic apparatus according to Example 1. A radiographic apparatus 9 according to Example 1 includes a gantry 11 having an opening for inserting a breast B of a subject M in a z-direction, and a detector ring 12 in a ring shape that is provided inside the gantry 11 for inserting the breast B of the subject M in the z-direction. The opening provided in the detector ring 12 has a cylinder shape (correctly, equilateral octagonal prism) extending in the z-direction. Consequently, the detector ring 12 self itself also extends in the z-direction. Here, a region of the opening of the detector ring 12 corresponds to a field of view where the radiographic apparatus 9 can generate a tomographic image P. The z-direction extends along a direction where the central axis of the detector ring 12 extends.

A top board 10 is provided for supporting the subject M with the abdomen placed thereon. The top board 10 includes a hole for inserting the breast B of the subject M in the z-direction, through which the breast B is inserted into inside of the detector ring 12. The opening of the gantry 11 is directed vertically upward. The breast B is to be inserted into the opening vertically downward.

The gantry 11 is placed on a support base 25. The support base 25 is behind the gantry 11 when seen from the subject M.

A shield plate 13 is made from tungsten, lead or the like. See Figure 1. Radiopharmaceutical also exists in portions other than the breast B of the subject M, from which portions annihilation gamma-ray pairs are generated. Annihilation gamma-ray pairs that are generated in the portions other than such region of interest enter into the detector ring 12. The annihilation gamma-ray pairs are obstructive for tomography. Then, the shield plate 13 in a ring shape is provided so as to cover one end of the detector ring 12 closer to the subject M in the z-direction. Since the shield plate 13 absorbs gamma-rays, gamma-rays emitted from the trunk of the subject M are to be absorbed into the shield plate 13. The shield plate 13 is provided so as to cover a side face of the detector ring 12 perpendicular to the central axis of the detector ring 12.

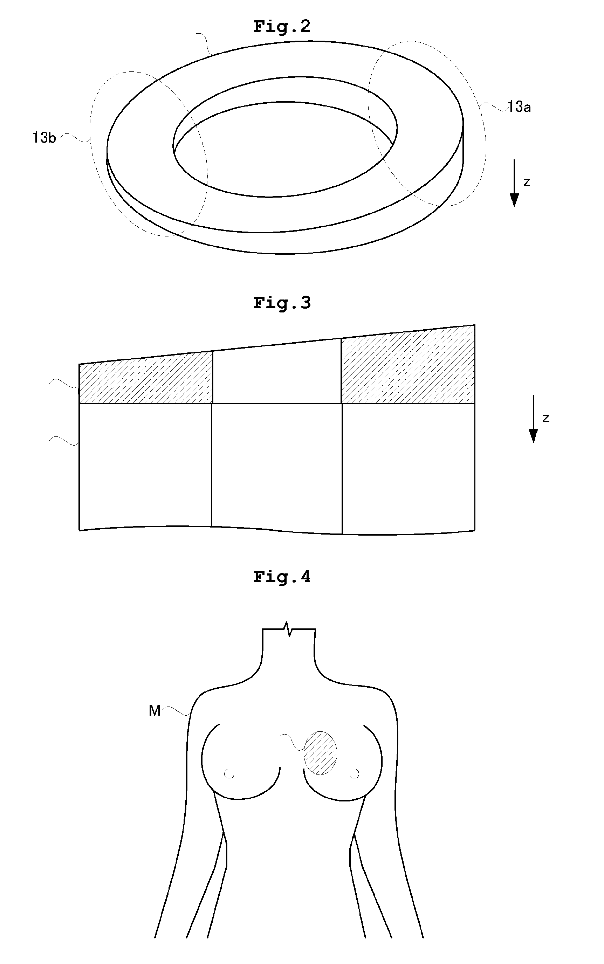

The shape of the shield plate 13 is the most characteristic feature in this invention. Figure 2 illustrates a schematic view of the shield plate 13. The shield plate 13 has a ring shape along the shape of the detector ring 12. The shield plate 13 has a thickness in its central axis direction (i.e., z-direction) that varies depending on portions thereof. The shield plate 13 has a thick portion 13a that is thick in the z-direction, and a thin portion 13b that is thin in the z-direction. One end of the shield plate 13 on the subject side is provided with an opening in an elliptical plane, and thus is planar. The breast B of the subject M is introduced into the opening. The thick portion 13a has a thickness of approximately 5mm, and the thin portion 13b has a thickness of approximately 1mm. In this way, the shield plate 13 is tapered.

Figure 3 illustrates a positional relationship between the shield plate 13 and the detector ring 12. The ring-shaped shield plate 13 is provided on the side face of the detector ring 12 such that the opening of the detector ring 12 may extend. The shield plate 13 is fixed on the detector ring 12.

A detector-ring rotating mechanism 23 rotates the detector ring 12 about the central axis. See Figure 1. The shield plate 13 is fixed on the detector ring 12. Consequently, the detector-ring rotating mechanism 23 rotates the shield plate 13 integrally with the detector ring 12. Operation of the detector-ring rotating mechanism 23 can shift the thick portion 13a of the shield plate 13 relative to the subject M. A detector-ring rotation controller 24 controls the detector-ring rotating mechanism 23. The detector-ring rotating mechanism 23 corresponds to the rotating device in this invention. The detector-ring rotation controller 24 corresponds to the rotation controller in this invention.

Next, description will be given of a positional relationship between the subject M and the shield plate 13 upon screening. A heart of the subject M is denoted by the reference h in Figure 4. Radiopharmaceutical tends to be accumulated in the heart. Consequently, relatively strong gamma-rays are emitted from the heart of the subject M into which radiopharmaceutical has been injected.

When gamma-rays from the heart are detected in the detector ring 12, it becomes difficult to screen the breast accurately. That is because gamma-rays generated from portions other than the breast are obstructive for imaging of radiopharmaceutical distribution in the breast B. Then the shield plate 13 is arranged in the direction where the thick portion 13a approaches the heart of the subject M upon screening.

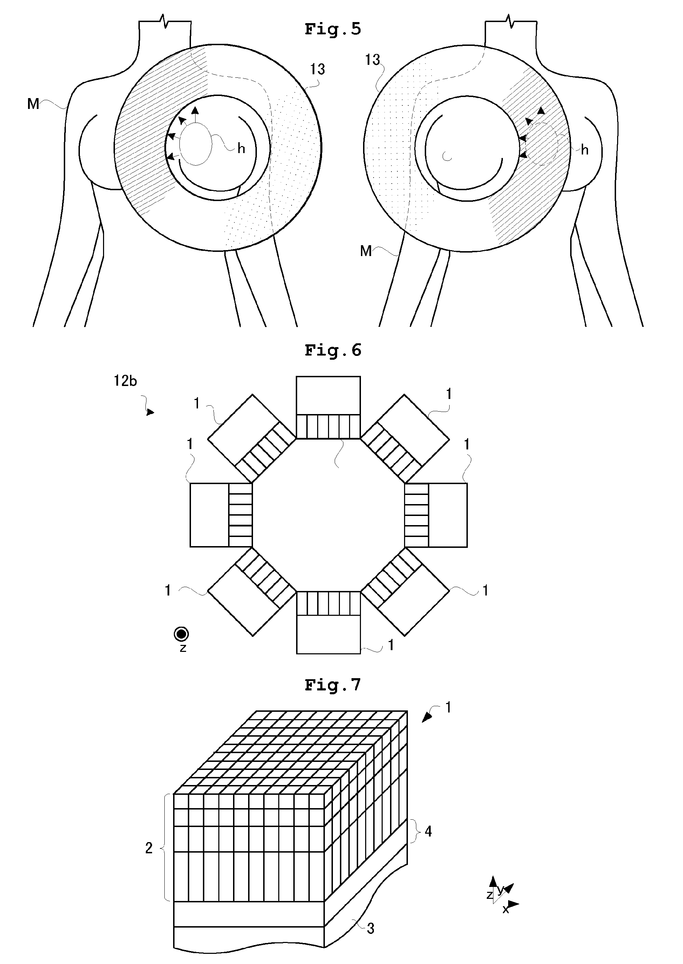

Figure 5 illustrates the shield plate 13 upon screening. When a diagnosis of a left breast of the subject M is conducted, the shield plate 13 rotates to a position as shown in Figure 5 on the left side, shown by slashes in the drawing, where the thick portion 13a approaches the heart h of the subject M. Then, radiation, shown by arrows in the drawing, that is emitted from the heart h is absorbed in the thick portion 13a of the shield plate 13. On the other hand, at the thin portion 13b of the shield plate 13, shown by half-tone dot meshing in the drawing, the detector ring 12 is closer to the subject M by a difference in thickness of the shield plate 13. Thereby the breast B of the subject M can be inserted deeply into the detector ring 12. Here, the heart h is located inside of the opening of the shield plate 13 when seen from the z-direction. The detector-ring rotating mechanism 23 rotates the shield plate 13.

Moreover, when a diagnosis of a right breast of the subject M is conducted, the shield plate 13 rotates to a position, shown by slashes in the drawing, where the thick portion 13a approaches the heart h of the subject M. See the right side of Figure 5. Then, radiation shown by arrows in the drawing, emitted from the heart h is absorbed in the thick portion 13a of the shield plate 13. On the other hand, at the thin portion 13b of the shield plate 13, shown by half-tone dot meshing in the drawing, the detector ring 12 is closer to the subject M by a difference in thickness of the shield plate 13. Thereby the breast B of the subject M can be inserted deeply into the detector ring 12. Here, the heart h is located in a portion covered with the thick portion 13a of the shield plate 13. The detector-ring rotating mechanism 23 rotates the shield plate 13.

Thus, when a diagnosing of either the right or left breast B is conducted, gamma-rays emitted from the trunk of the subject M is absorbed reliably in the thin portion 13b of the shield plate 13. Since an emission source, such as the heart, that emits many gamma-rays is not close to the thin portion 13b of the shield plate 13, a few gamma-rays enter into the thin portion 13b. Consequently, this portion can reliably absorb gamma-rays derived from the trunk when the shield plate 13 is thin. Moreover, the thick portion 13a of the shield plate 13 absorbs many gamma-rays reliably that are emitted from the heart h of the subject M. That is because gamma-rays that are to reach the detector ring 12 are reliably absorbed in a thick member that constitutes the thick portion 13a. As noted above, gamma-rays emitted from a portion, such as the heart, where radiopharmaceutical is accumulated, are absorbed into the thick portion 13a of the shield plate 13 and thus do not reach the detector ring 12. On the other hand, in the thin portion 13b, the breast B can be placed more tightly relative to the detector ring 12. Thus, gamma-rays emitted from the breast B reach the detector ring reliably.

For a diagnosis of the left breast of the subject M as illustrated in Figure 5 on the left side, gamma-rays emitted from the heart h of the subject M also travel toward the thin portion 13b of the shield plate 13. Here, since the thin portion 13b of the shield plate 13 is away from the heart h, gamma-rays reaching the thin portion 13b have small density. Consequently, gamma-rays emitted toward the thin portion 13b can be absorbed reliably when the thin portion 13b is formed of a thin member.

Description will be given of a construction of the detector ring 12. Eight radiation detectors 1 are arranged along an imaginary circle on a plane perpendicular to the z-direction (i.e., central axis direction), whereby one unit ring 12b is formed. Three unit rings 12b are arranged in the z-direction, whereby a detector ring 12 is formed. Specifically, see Figure 6.

Next, simple description will be given of a configuration of the radiation detector 1. Figure 7 is a perspective view illustrating a configuration of the radiation detector according to Example 1. As shown in Figure 7, the radiation detector 1 includes scintillators 2 that convert radiation into light, and a light detector 3 that is composed of photomultiplier tubes for detecting light. A light guide 4 is provided between the scintillators 2 and the light detector 3 for receiving light.

The scintillator 2 has two or more scintillation counter crystals arranged in a three-dimensional array. Each of the scintillation counter crystals is composed of Ce-doped Lu_{2(1-X)}Y_{2X}SiO₅ (hereinafter referred to as LYSO.) The light detector 3 allows determination of a light generating position about which scintillation counter crystal emits light. The light detector 3 also allows determination of intensity of light and time when light is generated. Here, the scintillator 2 that configures Example 1 is only exemplification of an aspect that may be adopted. Consequently, the configuration of this invention is not limited to this.

A clock 19 sends out time information represented by serial numbers to the detector ring 12. Time information about when gamma-rays were detected is given to detection signals outputted from the detector ring 12, and the detection signals are inputted into a filter 20 mentioned later.

The detection signals outputted from the detector ring 12 via a filter 20 mentioned later have been sent to a coincidence unit 21 (see Figure 1.) Two gamma-rays coincidently entering into the detector ring 12 is an annihilation gamma ray-pair derived from radiopharmaceutical within the subject. The coincidence unit 21 counts the frequency of detecting annihilation gamma-ray pairs for every combination of two scintillation counter crystals that form the detector ring 12. The resultant is sent to a tomographic-image generating section 22. Here, the coincidence unit 21 determines coincident property of the detection signals using the time information that the clock 19 gives to the detection signals.

The filter 20 is provided in order to prevent unnecessary data in the detector ring 12 from being sent to the coincidence unit 21. The filter 20 can filter the detection signals as to reduce load applied to the coincidence device 21.

The tomographic-image generating section 22 generates a tomographic image P when the opening of the detector ring 12 is cut in a plane in accordance with coincidence data outputted from the coincidence unit 21. The tomographic-image generating section 22 may receive data representing rotation of the detector ring 12 from a detector-ring rotation controller 24 to rotate a stereoscopic image of the breast B suitably, thereby generating a tomographic image P.

A display unit 36 is provided for displaying the tomographic image P generated by the tomographic-image generating section 22. A storage 37 stores all data generated through operation of each section and parameters referred to upon operation of each section. Examples of the data include the detection signals that the detector ring 12 outputs, the coincidence data that the coincidence unit 21 generates, and the tomographic image P.

The radiographic apparatus 9 includes a main controller 41 for performing an overall control of each section. The main controller 41 has a CPU, and provides each section 19, 20, 21, 22, 24 by executing various programs. The above sections may each be divided into a controller that performs their functions.

### <Operation of Radiographic Apparatus>

Next, description will be given of operation of the radiographic apparatus. For conducting a diagnosis of the breast B using the radiographic apparatus in Example 1, the detector ring 12 and the shield plate 13 firstly rotate. The subject M with radiopharmaceutical administered thereto is supported on the top board 10 with the abdomen placed thereon. Here, the thick portion 13a of the shield plate 13 is located close to the heart of the subject M.

When an operator instructs start to take a tomographic image via a console 35, the detector ring 12 starts detection of an annihilation gamma-ray pair (Imaging Starting Step S3.) The tomographic-image generating section 22 generates a tomographic image P through imaging the detected annihilation gamma-ray pairs. The tomographic image P is displayed on the display unit 36, whereby operation of the radiographic apparatus in Example 1 is completed.

As noted above, the shield plate 13 in this invention has a thickness that varies depending on portions thereof. That is, the shield plate 13 in this invention has a thick portion 13a that is thick in the central axis direction of the detector ring 12 (i.e., z-direction), and a thin portion 13b that is thin in the z-direction. In such thick portion 13a of the shield plate 13, gamma-rays emitted a lot from a heart of the subject, etc. can be absorbed reliably. Thereby, gamma-rays emitted from a portion, such as a heart, where radiopharmaceutical is accumulated are absorbed into the thick portion 13a of the shield plate 13 and thus do not reach the detector ring 12. On the other hand, in the thin portion 13b, the detector ring 12 can approach more tightly to the subject M, thereby the breast B can be placed more closely relative to the detector ring 12. Thus, gamma-rays emitted from the breast B reach the detector ring 12 reliably. As mentioned above, the radiographic apparatus 9 according to this invention can prevent gamma-rays emitted from the trunk of the subject M from entering into the detector ring 12 reliably. Moreover, the apparatus according to this invention can reliably insert the breast B into the detector ring 12 and conduct a reliable diagnosis of the breast B. Therefore, the apparatus according to this invention can achieve suppression of a false image, thereby obtaining a clear tomographic image P containing an entire image of the breast.

The aforementioned shield plate 13 has one end face on the subject side that is planar with no projection and depression. Thus the subject M does not contact to projections and depressions, which can achieve diagnosis without causing any pain to the subject M.

When the shield plate 13 is arranged in the direction where the thick portion 13a approaches the heart upon screening, a clear tomographic image P can be obtained with high reliability. Here, radiopharmaceutical tends to be accumulated in the heart of the subject M. Consequently, strong gamma-rays are emitted from the heart. With the foregoing construction, gamma-rays from the heart can be absorbed in the thick portion 13a of the shield plate 13 reliably. Thereby radiography of the breast B can be conducted with low noise.

The shield plate 13 can rotate, and accordingly the thick portion 13b can be moved to a portion in the trunk of the subject M where many gamma-rays are generated. Moreover, the thick portion 13a of the shield plate 13 may be shifted upon screening the right breast and the left breast of the subject M. Such configuration can cause the shield plate 13 to be shifted in accordance with a position of the trunk of the subject M that varies for every screening.

This invention is not limited to the foregoing configuration, but may be modified as follows.
(1) In the foregoing construction, the shield plate 13 is fixed on the detector ring 12. The detector ring 12 rotates, and accordingly the shield plate 13 rotates. This invention is not limited to this construction. Alternatively, the shield plate 13 can rotate relative to the detector ring 12. In this construction as shown in Figure 8, a shield-plate rotating mechanism 27 is provided instead of the detector-ring rotating mechanism 23, and a shield-plate rotation controller 28 is provided instead of the detector-ring rotation controller 24. A clearance is provided between the shield plate 13 and the detector ring 12 for varying a positional relationship to each other.
(2) In the foregoing construction, the shield plate 13 is tapered. This invention is not limited to the construction. Alternatively, as shown in Figure 9, the shield plate 13 may partially have a thick portion. Here, a protrusion 13c provided on the shield plate 13 corresponds to the thick portion in this invention, and a portion other than the protrusion 13c corresponds to the thin portion in this invention.
(3) In each of the foregoing examples, the scintillation counter crystal is composed of LYSO. Alternatively, the scintillation counter crystal may be composed of other materials, such as LGSO (Lu₂₍₁₋ₓ₎G₂ₓSiO₅) and GSO (Gd₂SiO₅), may be used in this invention. According to this modification, a method of manufacturing a radiation detector may be provided that allows provision of a radiation detector of low price.
(4) The light detector in the foregoing example is formed of the photomultiplier tube. This invention is not limited to this. A photodiode, an avalanche photodiode, a semiconductor detector, etc., may be used instead of the photomultiplier tube.

It will be apparent to those skilled in the art that various modifications and variations can be made in the present invention without departing from the spirit or scope of the invention. Thus, it is intended that the present invention cover the modifications and variations of this invention provided they come within the scope of the appended claims and their equivalents.

## Claims

1. A radiographic apparatus for breast examination, comprising:
a detector ring having detectors for detecting radiation being arranged in an arc shape; and
a shield plate for absorbing radiation that is provided as to cover a side face of the detector ring perpendicular to a central axis of the detector ring, the shield plate having a thick portion that is thick in a central axis direction and a thin portion that is thin in the central axis direction.

2. The radiographic apparatus for breast examination according to claim 1, wherein
one end face of the shield plate on a subject side is planar.

3. The radiographic apparatus for breast examination according to claim 1, wherein
the shield plate is arranged in a direction where the thick portion approaches the heart of the subject upon screening.

4. The radiographic apparatus for breast examination according to claim 1, further comprising:
a rotating device for rotating the shield plate about the central axis; and
a rotation control device for controlling the rotating device.

5. The radiographic apparatus for breast examination according to claim 2,
wherein
the shield plate is arranged in a direction where the thick portion approaches the heart of the subject upon screening.

6. The radiographic apparatus for breast examination according to claim 2, further comprising:
a rotating device for rotating the shield plate about the central axis; and
a rotation control device for controlling the rotating device.

7. The radiographic apparatus for breast examination according to claim 3, further comprising:
a rotating device for rotating the shield plate about the central axis; and
a rotation control device for controlling the rotating device.
